# EUROPÄISCHE PATENTSCHRIFT

(11) **EP 0 557 715 B1**
(45) Veröffentlichungstag und Bekanntmachung des Hinweises auf die Patenterteilung: **13.08.1997**
(21) Anmeldenummer: 93100850.2
(22) Anmeldetag: 21.01.1993
(51) Int. Cl.: G01N 33/49, B01L 3/14

(54) **Messgefäss**
Measuring vessel
Cuvette de mesure

(30) Priorität: 25.02.1992 DE 4205618
(43) Veröffentlichungstag der Anmeldung: 01.09.1993
(73) Patentinhaber: HEINRICH AMELUNG GmbH, D-32657 Lemgo (DE)
(72) Erfinder:
(74) Vertreter: Stracke, Alexander, Dipl.-Ing.

(56) Entgegenhaltungen:
- EP-A- 0 369 168
- DE-A- 2 937 195

## Beschreibung

Die vorliegende Erfindung betrifft ein Meßgefäß zur Aufnahme von zu untersuchenden Flüssigkeiten wie Blut, Blutplasma oder dergleichen gemäß dem Oberbegriff des Anspruchs 1.

Ein derartiges Meßgefäß ist aus der DE-PS 29 37 195 bekannt.

Mit dem darin gezeigten und beschriebenen Meßgefäß läßt sich eine sehr genaue mechanische Bestimmung des Gerinnungsverhaltens von Blut durchführen.

Neben dieser Untersuchungsmethode findet vielfach auch ein Verfahren Verwendung, das auf photometrischem Wege bestimmte Messungen von Blut, Blutplasma oder dergleichen ermöglicht.

Da hierzu ein einwandfreier Durchgang des von einem Sender ausgehenden Lichtes durch die Wandung des Meßgefäßes zwingend erforderlich ist, ist für die photometrischen Bestimmungen das gattungsgemäße Meßgefäß aufgrund seiner zylindrischen Form nicht geeignet.
Durch die Krümmung der Wandung werden die das Meßgefäß durchdringenden Lichtstrahlen abgelenkt und würden so im Einsatzfall zu ungenauen Meßergebnissen führen.

Des weiteren läßt sich mit dem bekannten Meßgefäß ein automatischer Untersuchungsablauf nur sehr schwer und unvollkommen realisieren. Insbesondere für eine photometrische Messung ist die zylindrische Form des Meßgefäßes ungeeignet, da eine exakte Positionierung zum Sender Voraussetzung für eine einwandfreie Messung ist.

Aber nicht nur einer automatischen Zuführung steht die zylindrische Form als hinderlich entgegen, sondern auch einer optimalen Deponierung in einem Vorratsbehälter, der herstellerseitig mit einer Vielzahl von Meßgefäßen bestückt und komplett einem Automaten des Verwenders zugeführt wird, in dem dann das jeweilige Meßgefäß automatisch aus dem Vorratsbehälter entnommen wird. Dabei kommt es auch darauf an, den Deponieraum des Vorratsbehälters optimal auszunutzen, d.h., eine größtmögliche Anzahl von Meßgefäßen unterzubringen.
Naturgemäß ist hierzu eine zylindrische Form nicht geeignet.

Der vorliegenden Erfindung liegt daher die Aufgabe zugrunde, ein Meßgefäß der gattungsgemäßen Art so zu gestalten, daß sowohl eine mechanische Messung nach der Kugelmethode als auch eine photometrische Messung der zu untersuchenden Flüssigkeiten möglich ist und daß bei optimaler Ausnutzung eines Vorratsbehälters eine automatische Zuführung in ein Untersuchungsgerät möglich ist.

Diese Aufgabe wird erfindungsgemäß durch die im kennzeichnenden Teil des Anspruchs 1 genannten Merkmale gelöst.

Mit dem derart ausgestatteten Meßgefäß können nun photometrische Analysen über den gesamten Spektralbereich durchgeführt werden, insbesondere für chromogene Substrate, enzymkinetische und Endpunktbestimmungen.

Während zumindest zwei sich gegenüberliegende, parallele Seitenflächen sich über nahezu die gesamte Länge des Rohrteiles erstrecken, ist der an den Boden angrenzende Seitenbereich des Rohres zylindrisch ausgeformt.

So kann der Bereich, der oberhalb des Begrenzungszapfens liegt, für photometrische Untersuchungen genutzt werden. Der untere Bodenbereich hingegen wird für die Untersuchung nach der Kugelmethode eingesetzt.

Neben der kombinierten Verwendungsfähigkeit des neuen Meßgefäßes in bezug auf die verschiedenen Meßmethoden, bietet das Meßgefäß den weiteren Vorteil, daß es für einen automatischen Untersuchungsablauf eingesetzt werden kann.

Die rechteckige Grundrißausbildung ermöglicht auch eine optimale Anordnung in einem Vorratsbehälter, in dem eine Vielzahl von den im wesentlichen quaderförmigen Meßgefäßen gelagert sind und der für eine automatische Zuführung der Meßgefäße in einem Untersuchungsautomaten zu plazieren ist. Dabei liegen jeweils zwei Vorratsbehälter an den einander zugewandten planebenen Anlageflächen an, die durch die parallel zueinander verlaufenden Breitseiten des Schachtes gebildet werden.

Durch die rechteckige Grundrißgestaltung des Vorratsbehälters einerseits und durch das vorgesehene Fußelement, das nach einer vorzugsweisen Ausgestaltung des Meßgefäßes aus Fußflächen gebildet wird, die sich seitlich des Rohrteiles in Verringerung der Breitseiten des Schachtes erstrecken, wird sowohl ein lagegenaues wie auch ein sicheres Herausschieben des Meßgefäßes aus dem Vorratsbehälter ermöglicht, wobei durch das Fußelement ein sicherer Halt beim Herausschieben gewährleistet wird.

In bekannter Weise besteht das Meßgefäß aus einem lichtdurchlässigen Kunststoff. Da sich aus Fertigungsgründen der Anguß an der Außenseite des Bodens des Meßgefäßes befindet, ist es wichtig, daß sich die Fußflächen geringfügig über den Boden hinaus erstrecken, so daß es durch den Anguß nicht zu Verhakungen beim Schieben aus dem Vorratsbehälter kommt.

Zur besseren Abstützung der Meßgefäße untereinander ist nach einem weiteren Gedanken der Erfindung vorgesehen, daß die Fußflächen planeben mit den Breitseiten verlaufen, so daß sich praktisch über die gesamte Breitseite des Meßgefäßes eine Anlagefläche bildet.

Im übrigen ist es für einen störungsfreien automatischen Arbeitsablauf wichtig, daß das Meßgefäß symmetrisch ausgebildet ist.
Weitere vorteilhafte Ausgestaltungen der Erfindung sind in den Unteransprüchen gekennzeichnet.
Ein Ausführungsbeispiel der Erfindung wird nachfolgend anhand der beigefügten Zeichnungen beschrieben.
Es zeigen:

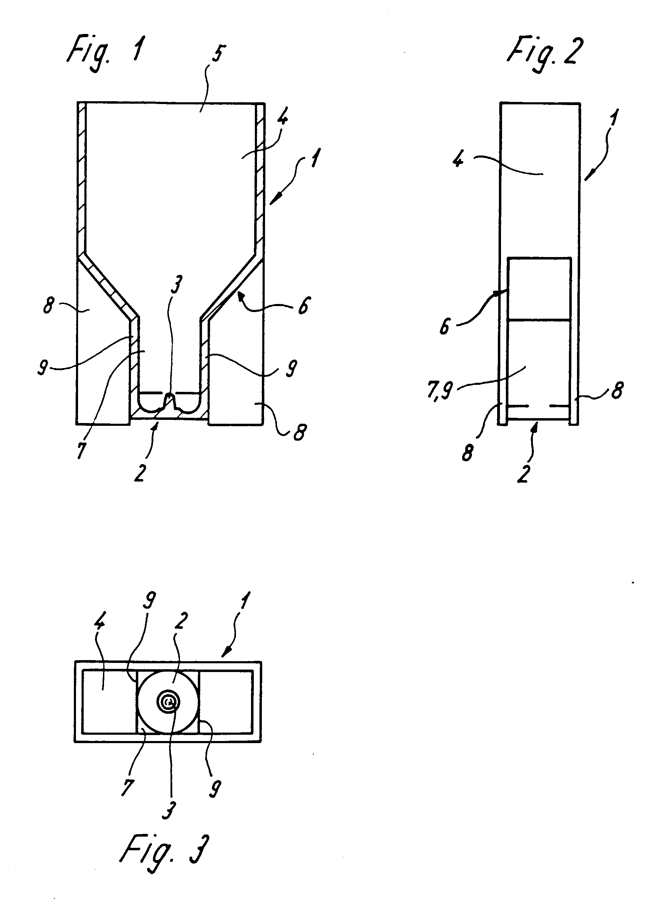

- Fig. 1: einen Längsschnitt durch ein in Vorderansicht dargestelltes Meßgefäß,
- Fig. 2: eine Seitenansicht des Maßgefäßes,
- Fig. 3: eine Draufsicht auf das Meßgefäß.

Ein in den Figuren insgesamt mit dem Bezugszeichen 1 versehenes Meßgefäß ist in seiner Außenform etwa quaderförmig ausgebildet und weist einen im Querschnitt rechteckigen Schacht 4 auf, dessen eines Ende die Einlaßöffnung 5 des Meßgefäßes 1 bildet, durch das eine zu untersuchende Flüssigkeit einführbar ist.

Der der Einlaßöffnung 5 gegenüberliegende Endbereich ist als Trichter 6 ausgebildet und mündet in ein Rohrteil 7, das durch einen Boden 2 verschlossen ist.

Zentrisch daran angeformt ist ein nach innen ragender Begrenzungszapfen 3.

Das Rohrteil 7 ist in vorliegenden Ausführungsbeispiel im Querschnitt quadratisch ausgebildet, wobei die sich gegenüberliegenden Seitenflächen 9 parallel zueinander verlaufen und sich im Innenbereich nicht ganz über die gesamte Länge erstrecken. Im unteren, dem Boden 2 zugeordneten Endbereich ist das Rohrteil 7 zylindrisch ausgeformt. Der Boden 2 ist zwischen dem Begrenzungszapfen 3 und der umlaufenden, zylindrischen Seitenwandung muldenförmig gestaltet und bildet so die Laufbahn für eine nicht dargestellte Kugel, die der Blutgerinnungs-Messung nach der Kugelmethode dient.

Der lichte Durchmesser des Bodens 2 bzw. des zylindrischen Endbereiches des Rohrteiles 7 entspricht der lichten Breite des gesamten Meßgefäßes 1, so daß auch die lichten Seitenlängen des im Querschnitt quadratischen Rohrteiles 7 diesem Abmaß entsprechen.

Die erhöhte Standfestigkeit des Meßgefäßes, die insbesondere beim automatischen Transport in einem Untersuchungsautomaten sehr wichtig ist, wird durch angeformte Fußflächen 8 erreicht.
Dabei sind diese Fußflächen auf beiden Breitseiten des Meßgefäßes 1 vorgesehen, wobei jeweils rechts und links vom Rohrteil 7 angeordnete Fußflächen 8 gemeinsam mit der zugeordneten Seitenfläche des Rohrteiles 7 und der sich nach oben hin anschließenden Seitenfläche des Schachtes 4 eine Breitseite des Meßgefäßes 1 bilden.

### Bezugszeichen

- 1: Meßgefäß
- 2: Boden
- 3: Begrenzungszapfen
- 4: Schacht
- 5: Einlaßöffnung
- 6: Trichter
- 7: Rohrteil
- 8: Fußflächen
- 9: Seitenflächen

## Patentansprüche

1. Meßgefäßzur Aufnahme von zu untersuchenden Flüssigkeiten wie Blut, Blutplasma o.dgl., mit einem in dessen kreisrundem Bodenbereich zentrisch angeordneten, vorstehenden Begrenzungszapfen (3),wobei der Boden (2) die Laufbahn für eine Kugel bildet, mit deren Hilfe das Gerinnungsverhalten von Blut meßbar ist, **dadurch gekennzeichnet,** daß das Meßgefäß (1) einen im Querschnitt rechteckigen Schacht (4) aufweist, dessen eines Ende die Einlaßöffnung (5) bildet, während der gegenüberliegende Endbereich trichterförmig ausgebildet ist und in ein Rohrteil (7) mündet, das zumindest zwei parallele, sich über nahezu die gesamte Länge erstreckende Seitenflächen (9) aufweist und das endseitig durch den Boden (2) verschlossen ist, wobei die parallel zueinander verlaufenden Breitseiten des Schachtes (4) jeweils eine planebene Anlagefläche bilden, und daß mit dem Rohrteil (7) mindestens ein Fußelement verbunden ist, auf das das Meßgefäß (1) aufstellbar ist.

2. Meßgefäß nach Anspruch 1, dadurch gekennzeichnet, daß dies symmetrisch ausgebildet ist.

3. Meßgefäß nach Anspruch 1, dadurch gekennzeichnet, daß das Rohrteil (7) im Querschnitt quadratisch ist.

4. Meßgefäß nach Anspruch 1 oder 3, dadurch gekennzeichnet, daß der lichte Durchmesser des Bodens (2) bzw. der sich daran anschließenden zylindrischen Seitenwandung des Rohrteiles (7) dessen lichter Seitenlänge entspricht.

5. Meßgefäß nach Anspruch 1, dadurch gekennzeichnet, daß als Fußelemente seitlich des Rohrteiles (7) in Verlängerung der Breitseiten des Schachtes (4) sich erstreckende Fußflächen (8) vorgesehen sind.

6. Meßgefäß nach Anspruch 5, dadurch gekennzeichnet, daß sich die Fußflächen (8) bis zu den Außenkanten der Breitseiten des Schachtes (4) erstrecken.

7. Meßgefäß nach Anspruch 5 oder 6, dadurch gekennzeichnet, daß die Fußflächen (8) geringfügig über den Boden (2) hinausragen.

8. Meßgefäß nach Anspruch 5, dadurch gekennzeichnet, daß die Fußflächen (8) planeben mit den Breitseiten verlaufen.

## Claims

1. A measuring vessel for receiving liquids to be tested such as blood, blood plasma or the like, comprising a projecting delimitation spigot (3) arranged centrally in the circular bottom region thereof, wherein the bottom (2) forms the track for a ball, by means of which the coagulation characteristics of blood can be measured, characterised in that the measuring vessel (1) has a shaft portion (4) of rectangular cross-section, of which one end forms the inlet opening (5) while the oppositely disposed end region is of a funnel-shaped configuration and opens into a tubular portion (7) which has at least two parallel side surfaces (9) extending over almost the entire length and which is closed at the end by the bottom (2), wherein the mutually parallel wide sides of the shaft portion (4) each form a respective planar contact surface, and that connected to the tubular portion (7) is at least one foot element on to which the measuring vessel (1) can be set up.

2. A measuring vessel according to claim 1 characterised in that it is of a symmetrical configuration.

3. A measuring vessel according to claim 1 characterised in that the tubular portion (7) is of a square cross-section.

4. A measuring vessel according to claim 1 or claim 3 characterised in that the inside diameter of the bottom (2) or the adjoining cylindrical side wall of the tubular portion (7) corresponds to the internal side length thereof.

5. A measuring vessel according to claim 1 characterised in that foot surfaces (8) extending laterally of the tubular portion (7) as prolongations of the wide sides of the shaft portion (4) are provided as foot elements.

6. A measuring vessel according to claim 5 characterised in that the foot surfaces (8) extend as far as the outside edges of the wide sides of the shaft portion (4).

7. A measuring vessel according to claim 5 or claim 6 characterised in that the foot surfaces (8) project slightly beyond the bottom (2).

8. A measuring vessel according to claim 5 characterised in that the foot surfaces (8) extend in planar relationship with the wide sides.

## Revendications

1. Récipient mesureur destiné à contenir des liquides à analyser, tels que du sang, du plasma sanguin ou similaire, comportant un téton séparateur (3) faisant saillie, situé au centre de sa zone formant fond arrondie en forme circulaire, le fond (2) constituant la zone de roulement d'une bille au moyen de laquelle la coagulation du sang peut être mesurée, caractérisé en ce que le récipient mesureur (1) présente une cuve (4) de section rectangulaire, dont une extrémité constitue l'orifice d'entrée (5), tandis que la zone terminale opposée présente une forme d'entonnoir et débouche dans une partie tubulaire (7), qui présente au moins deux faces latérales (9) parallèles, s'étendant sur presque toute sa longueur, et qui est fermée, du côté terminal, par le fond (2), les côtés plats de la cuve (4), parallèles les uns aux autres, constituant chacun une surface d'appui plane, et en ce qu'à la partie tubulaire (7) est relié au moins un élément formant pied, sur lequel le récipient mesureur (1) peut être posé.

2. Récipient mesureur selon la revendication 1, caractérisé en ce que celui-ci est conformé de façon symétrique.

3. Récipient mesureur selon la revendication 1, caractérisé en ce que la partie tubulaire (7) est de section carrée.

4. Récipient mesureur selon la revendication 1 ou 3, caractérisé en ce que le diamètre intérieur du fond (2) et/ou de la paroi latérale cylindrique de la partie tubulaire (7) qui lui fait suite correspond à sa longueur latérale intérieure.

5. Récipient mesureur selon la revendication 1, caractérisé en ce que, sur le côté de la partie tubulaire (7), en prolongement des côtés plats de la cuve (4), des embases (8) sont prévues pour servir d'éléments formant pieds.

6. Récipient mesureur selon la revendication 5, caractérisé en ce que les embases (8) s'étendent jusqu'aux arêtes extérieures des côtés plats de la cuve (4).

7. Récipient mesureur selon la revendication 5 ou 6, caractérisé en ce que les embases (8) dépassent légèrement par rapport au fond (2).

8. Récipient mesureur selon la revendication 5, caractérisé en ce que les embases (8) s'étendent sur le même plan que les côtés plats.
